(19) Europäisches Patentamt
European Patent Office
Office européen des brevets

(11) EP 0 868 168 B1

(12) **EUROPÄISCHE PATENTSCHRIFT**

(45) Veröffentlichungstag und Bekanntmachung des Hinweises auf die Patenterteilung:
**10.01.2001 Patentblatt 2001/02**

(21) Anmeldenummer: **96941601.5**

(22) Anmeldetag: **27.11.1996**

(51) Int Cl.[7]: **A61K 7/42**, A61K 7/48

(86) Internationale Anmeldenummer:
**PCT/EP96/05237**

(87) Internationale Veröffentlichungsnummer:
**WO 97/21421 (19.06.1997 Gazette 1997/26)**

(54) **KOSMETISCHE UND DERMATOLOGISCHE LICHTSCHUTZFORMULIERUNGEN IN FORM VON O/W-EMULSIONEN**

COSMETIC AND DERMATOLOGICAL SOLAR PROTECTION FORMULATIONS IN THE FORM OF O/W EMULSIONS

FORMULATIONS PHOTOPROTECTRICES COSMETIQUES ET DERMATOLOGIQUES SOUS FORME D'EMULSIONS HUILE DANS EAU

(84) Benannte Vertragsstaaten:
**AT BE CH DE ES FR GB IT LI NL SE**

(30) Priorität: **08.12.1995 DE 19545789**

(43) Veröffentlichungstag der Anmeldung:
**07.10.1998 Patentblatt 1998/41**

(73) Patentinhaber: **Beiersdorf Aktiengesellschaft
20245 Hamburg (DE)**

(72) Erfinder:
- **GERS-BARLAG, Heinrich
D-25495 Kummerfeld (DE)**
- **KRÖPKE, Rainer
D-22527 Hamburg (DE)**

(56) Entgegenhaltungen:
**WO-A-93/04666**
**WO-A-94/18940**
**FR-A- 2 268 516**
**US-A- 5 008 100**
**US-A- 5 041 281**
**US-A- 5 306 486**
**US-A- 5 498 406**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 868 168 B1

Printed by Jouve, 75001 PARIS (FR)

## Beschreibung

Kosmetische und dermatologische Lichtschutzformulierungen in Form von O/W- Emulsionen mit einem Gehalt an hydrophoben anorganischen Mikropigmenten und Isoalkansäuren

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schütze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

UV-Absorber bzw. UV-Reflektoren sind die meisten anorganischen Pigmente, die bekannterweise in der Kosmetik zum Schütze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes "Weißeln" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondern setzt sich während der Lagerung fort. Das "Weißeln" kann sich daher über einen längeren Zeitraum noch verstärken.

Ein weiterer Nachteil ist, daß es in entsprechenden Emulsionen, insbesondere O/W-Emulsionen, zu Migrationen anorganischer Partikel zwischen der Ölphase, wo ihre Gegenwart erwünscht ist, und der Wasserphase kommen kann. Finden sich aber in der Wasserphase derartige Partikel als Kristallisationskeime, so daß bestimmte Stoffe, unter anderem Emulgatoren, und von diesen insbesondere die Stearinsäure, auskristallisieren und dadurch die Stabilität der Emulsionen stark beeinträchtigen.

Auch kann das Ausölen oder gar Brechen einer Emulsion mittel- oder langfristig Folge einer derartigen Agglomeration sein.

Verschlimmernd kommt hinzu, daß sich auch die UV-Absorptions- bzw. UV-Reflexionseigenschaften einer Zubereitung verschlechtern, da diese Eigenschaften stark abhängig sind von der Dispersität der Partikel.

Ein weiterer Nachteil des Einsatzes anorganischer Pigmente in kosmetischen Formulierungen ist, daß solche Pigmente in den weitaus meisten Fällen zu starker Hauttrockenheit führen.

Aufgabe der vorliegenden Erfindung war mithin, den Nachteilen des Standes der Technik abzuhelfen.

Es hat sich überraschenderweise gezeigt, und darin liegt die Lösung der Aufgaben begründet, daß O/W-Emulsionen,

- enthaltend eine Wasserphase, gegebenenfalls übliche, in Wasser lösliche oder dispergierbare Substanzen,

- enthaltend eine Ölphase, in welcher ein oder mehrere hydrophobe anorganische Mikropigmente, in suspendierter Form vorliegen,
- enthaltend mindestens einen Emulgator, gewählt aus der Gruppe der verzweigten Alkancarbonsäuren.

den Nachteilen des Standes der Technik abhelfen.

Erfindungsgemäß ist auch die Verwendung von Emulgatoren, gewählt aus der Gruppe der verzweigten Alkancarbonsäuren in O/W-Emulsionen, diese

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,

zur Stabilisierung der O/W-Emulsion gegen die Migration der anorganischen Pigmente, insbesondere hydrophoben anorganischen Mikropigmente aus der Ölphase in die Wasserphase und die Folgen einer solchen Migration.

Zwar beschreibt die US-A-5,041,281 O/W-Emulsionen, welche auch anorganische Pigmente enthalten können. Dieses Dokument konnte indes nicht den Weg zur vorliegenden Erfindung ebnen.

Erfindungsgemäße anorganische Pigmente werden insbesondere vorteilhaft gewählt aus der Gruppe der Metalloxide und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter. R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, UVITAN 160 und UVITAN "&" von der Firma Kemira sowie T805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 20,0 Gew.-%, bevorzugt 0,5 - 10,0 Gew.-% gewählt, bezogen auf das Gesamtgewicht der Zubereitungen.

Die als Emulgatoren wirkenden verzweigten Alkancarbonsäuren weisen vorteilhaft 14 bis 24 Kohlenstoffatome auf. Bevorzugt handelt es sich dabei um iso-Alkancarbonsäuren

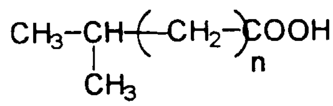

wobei n Zahlen von 10 bis 20 annimmt, und/oder antiso-Alkancarbonsäuren.

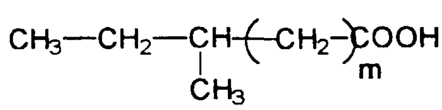

wobei m Zahlen von 9 bis 19 annimmt.

Bevorzugter Emulgator im Sinne der vorliegenden Erfindung ist die iso-Stearinsäure.

Es ist vorteilhaft, weitere übliche Emulgatoren in den erfindungsgemäßen O/W-Emulsionen zu verwenden.

Die erfindungsgemäßen Emulsionen sind vorteilhaft dadurch gekennzeichnet, daß der oder die Emulgatoren,

gewählt aus der Gruppe der verzweigten Alkancarbonsäuren, in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Es ist erfindungsgemäß vorteilhaft, in den erfindungsgemäßen Zubereitungen übliche öllösliche UVA-Filter und/oder UVB-Filter in der Lipidphase und/oder übliche wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Die UVB-Filter können öllöslich oder wasserlöslich sein. Vorteilhafte öllösliche UVB-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Ein vorteilhafter UVB-Filter, der vorteilhaft im Sinne der vorliegenden Erfindung verwendet werden kann, ist der 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

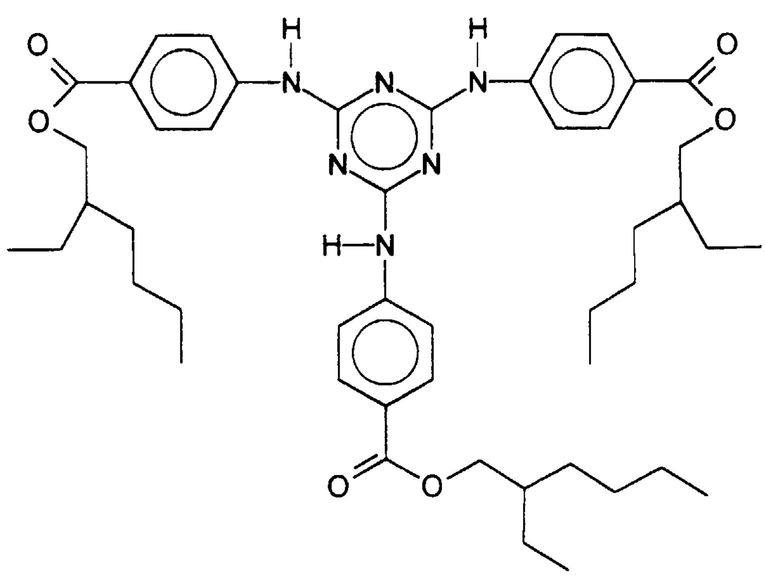

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Besonders vorteilhaft sind erfindungsgemäße Zubereitungen, welche als weitere UV-Schutzsubstanzen Sa-

licylsäurederivate 4-Isopropylbenzylsalicylat, 2-Ethylhexylsalicylat, (Octylsalicylat) und/oder Homomenthylsalicylat enthalten, insbesondere solcher Salicylsäurederivate der allgemeinen Struktur

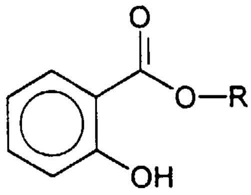

wobei R darstellen kann:

(1) einen verzweigten oder unverzweigten Alkylrest mit 5 - 18 Kohlenstoffatomen oder
(2) einen unsubstituierten Cycloalkylrest mit 5 - 7 Kohlenstoffatomen oder
(3) einen mit bis zu 10 verzweigten oder unverzweigten Alkylresten mit 5 - 18 Kohlenstoffatomen substituierten Cycloalkylrest mit 5 - 7 Kohlenstoffatomen oder
(4) einen unsubstituierten Phenylrest oder
(5) einen mit bis zu 5 verzweigten oder unverzweigten Alkylresten mit 5 - 18 Kohlenstoffatomen substituierten Phenylrest.

Zu gebräuchlichen UV-Filtern gehören darunter

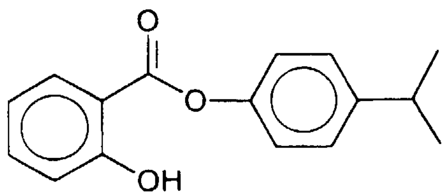

(4-Isopropylbenzylsalicylat),

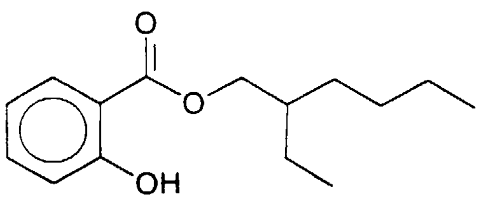

(2-Ethylhexylsalicylat, Octylsalicylat),

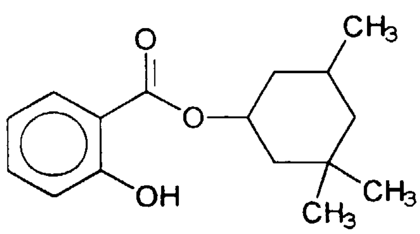

(Homomenthylsalicylat).

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter in den erfindungsgemäßen Zubereitungen einzusetzen, welche bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten

Mengen eingesetzt werden.

Ferner ist vorteilhaft, die erfindungsgemäßen Wirkstoffkombinationen mit weiteren UVA- und/oder UVB-Filtern zu kombinieren.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;

- Silikonöle wie Dimethylpolysiloxane, Diethylpolysiloxane, Diphenylpolysiloxane sowie Mischformen daraus.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen das Wesen der vorliegenden Erfindung näher umreißen, ohne die Erfindung einzuschränken.

**Beispiel 1**

| **Sonnenschutzemulsion** | |
|---|---|
| | Gew.-% |
| Glycerylstearat | 4,00 |
| Octyldodecanol | 10,00 |
| Isostearinsäure | 4,00 |
| Cetearylalkohol | 1,00 |
| Hydroxymethylpropylcellulose | 0,50 |
| Parsol MCX | 6,00 |
| hydrophobes $TiO_2$ | 8,00 |
| NaOH | 0,60 |
| Parfum, Farbstoffe, Konservierungsmittel | q.s. |
| Wasser | ad 100,00 |

**Beispiel 2**

| **Gesichtspflege mit UV-Schutz** | |
|---|---|
| | Gew.-% |
| Glycerylstearat | 1,50 |
| Isostearinsäure | 3,50 |
| Hydrierte Kokosnußfettsäuren | 2,00 |
| Myristylmyristat | 3,60 |
| Cetearylalkohol | 1,40 |
| Glycerin | 5,00 |
| hydrophobes $TiO_2$ | 3,50 |
| Ethanol | 5,00 |
| KOH | 0,30 |
| Parfum, Farbstoffe, Konservierungsmittel | q.s. |
| Wasser | ad 100,00 |

**Beispiel 3**

| Sonnenschutzemulsion mit hohem UV-Schutz | |
|---|---|
| | Gew.-% |
| Dicaprylylether | 6,00 |
| Jojobaöl | 1,50 |
| Vitamin E Acetat | 1,00 |
| Stearylalkohol | 1,50 |
| Isostearinsäure | 6,00 |
| Oxybenzon | 3,00 |
| Parsol 1789 | 2,00 |
| Parsol MCX | 8,00 |
| Methylpolyglycerylglucosedistearat | 1,50 |
| hydrophobes $TiO_2$ | 4,50 |
| Ethanol | 5,00 |
| KOH | 0,30 |
| Triethanolamin | 2,00 |
| Parfum, Farbstoffe, Konservierungsmittel | q.s. |
| Wasser | ad 100,00 |

**Patentansprüche**

1. O/W-Emulsionen,

   - enthaltend eine Wasserphase, gegebenenfalls übliche, in Wasser lösliche oder dispergierbare Substanzen,
   - enthaltend eine Ölphase, in welcher ein oder mehrere hydrophobe anorganische Mikropigmente, in suspendierter Form vorliegen,
   - enthaltend mindestens einen Emulgator, gewählt aus der Gruppe der verzweigten Alkancarbonsäuren.

2. O/W-Emulsionen nach Anspruch 1, dadurch gekennzeichnet, daß die als Emulgatoren wirkenden verzweigten Alkancarbonsäuren 14 bis 24 Kohlenstoffatome aufweisen.

3. O/W-Emulsionen nach Anspruch 2 dadurch gekennzeichnet, daß die als Emulgatoren wirkenden verzweigten Alkancarbonsäuren gewählt werden aus der Gruppe der Isoalkansäuren der allgemeinen Formel

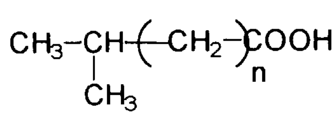

   wobei n Zahlen von 10 bis 20 annimmt, und/oder der anteiso-Alkancarbonsäuren der allgemeinen Formel

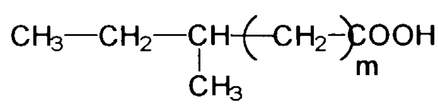

   wobei m Zahlen von 9 bis 19 annimmt.

4. O/W-Emulsionen nach Anspruch 1, dadurch gekennzeichnet, daß Titandioxidpigmente verwendet werden, und daß die hydrophobe Oberflächenschicht der Titandioxidpigmente nach einer Rektion gemäß

$$n\ TiO_2 + m\ (RO)_3\ Si\text{-}R' \rightarrow n\ TiO_2\ (\text{oberfl.})$$

erzeugt wird.

**5.** Verwendung von O/W-Emulsionen nach Anspruch 1 als Lichtschutzmittel.

**6.** Verwendung von Emulgatoren, gewählt aus der Gruppe der verzweigten Alkancarbonsäuren in O/W-Emulsionen, diese

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,

zur Stabilisierung der O/W-Emulsion gegen die Migration der anorganischen Pigmente, insbesondere hydrophoben anorganischen Mikropigmente aus der Ölphase in die Wasserphase und die Folgen einer solchen Migration.

## Claims

**1.** O/W emulsions

- comprising an aqueous phase and, optionally, customary substances which are soluble or dispersible in water,
- comprising an oil phase in which one or more hydrophobic inorganic pigments are present in suspended form,
- comprising at least one emulsifier selected from the group consisting of branched alkanecarboxylic acids.

**2.** O/W emulsions according to Claim 1, characterized in that the branched alkanecarboxylic acids which act as emulsifiers have from 14 to 24 carbon atoms.

**3.** O/W emulsions according to Claim 2, characterized in that the branched alkanecarboxylic acids which act as emulsifiers are selected from the group consisting of isoalkanoic acids of the general formula

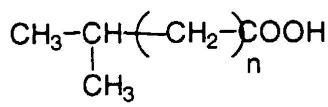

in which n assumes numbers from 10 to 20, and/or of the anteisoalkanecarboxylic acids of the general formula

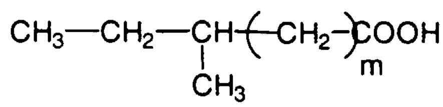

in which m assumes numbers from 9 to 19.

**4.** O/W emulsions according to Claim 1, characterized in that titanium dioxide pigments are used, and in that the hydrophobic surface layer of the titanium dioxide pigments is produced by a reaction according to

$$n\ TiO_2 + m\ (RO)_3Si\text{-}R'\ 6\ n\ TiO_2(\text{surface}).$$

**5.** Use of O/W emulsions according to Claim 1 as light protection agents.

**6.** Use of emulsifiers selected from the group consisting of branched alkanecarboxylic acids in O/W emulsions, the latter

- comprising an aqueous phase and, optionally, customary substances which are soluble or dispersible in water,

- comprising an oily phase in which one or more inorganic pigments, in particular hydrophobic inorganic micropigments, are present in suspended form,

for stabilizing the O/W emulsion against the migration of the inorganic pigments, in particular hydrophobic inorganic micropigments, from the oil phase into the aqueous phase and against the consequences of such a migration.

**Revendications**

1. Emulsions huile dans l'eau,

   - contenant une phase aqueuse, le cas échéant, des substances usuelles solubles ou dispersables dans l'eau;
   - contenant une phase d'huile, dans laquelle un ou plusieurs micropigments inorganiques hydrophobes sont présents sous forme de suspension;
   - contenant au moins un émulsionnant, choisi parmi le groupe des acides alcanecarboxyliques ramifiés.

2. Emulsions huile dans l'eau suivant la revendication 1, caractérisées en ce que les acides alcanecarboxyliques ramifiés agissant en tant qu'émulsionnants présentent 14 à 24 atomes de carbone.

3. Emulsions huile dans l'eau suivant la revendication 2, caractérisées en ce que les acides alcanecarboxyliques ramifiés agissant comme des émulsionnants sont choisis parmi le groupe des acides i-alcanoïques de formule générale

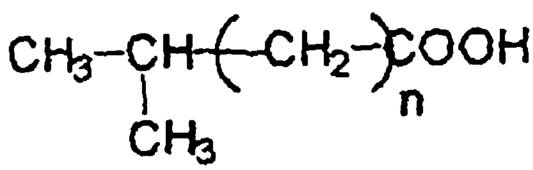

n signifiant des nombres de 10 à 20, et/ou des acides carboxyliques ante-i-alcanoïques de formule générale

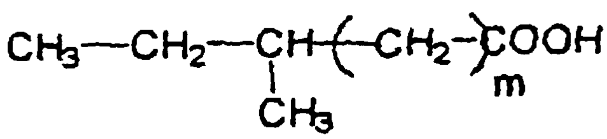

m signifiant des nombres de 9 à 19.

4. Emulsions huile dans l'eau suivant la revendication 1, caractérisées en ce que l'on utilise des pigments de dioxyde de titane et en ce que la couche de surface hydrophobe des pigments de dioxyde de titane est produite suivant la réaction suivante

$$n\ TiO_2 + m\ (RO)_3Si\text{-}R' \rightarrow n\ TiO_2(surf.)$$

5. Utilisation d'émulsions huile dans l'eau suivant la revendication 1, en tant qu'agent photoprotecteur.

6. Utilisation d'émulsionnants, choisis parmi le groupe des acides alcanecarboxyliques ramifiés dans des émulsions huile dans l'eau, celles-ci

   - contenant une phase aqueuse, le cas échéant, des substances usuelles solubles ou dispersables dans l'eau,
   - contenant une phase d'huile, dans laquelle un ou plusieurs pigments inorganiques, en particulier des micropigments inorganiques hydrophobes, sont présents sous forme de suspension,

pour stabiliser l'émulsion huile dans l'eau contre la migration des pigments inorganiques, en particulier des micropigments inorganiques hydrophobes, de la phase d'huile dans la phase aqueuse et contre les conséquences d'une

telle migration.